# EUROPEAN PATENT APPLICATION

(11) **EP 3 677 205 A1**
(43) Date of publication of application: **08.07.2020**
(21) Application number: 20150241.6
(22) Date of filing: 03.01.2020
(51) Int. Cl.: A61B 18/02, A61B 18/00

(54) **CRYOABLATION CATHETER FOR TREATMENT OF HYPERTROPHIC CARDIOMYOPATHY AND METHOD FOR TREATMENT USING SAME**

(30) Priority: 04.01.2019 KR 20190001332
(71) Applicant: Tau Pnu Medical Co., Ltd., Yangsan 609-836 (KR)
(72) Inventor: KIM, June-hong, 608-776 Busan (KR); PARK, Kyone Peter, 50653 Yangsan (KR); SUNG, Si-Chan, 609-836 Yangsan (KR)
(74) Representative: HGF

(57) **Abstract**

A cryoablation catheter for treatment of hypertrophic cardiomyopathy is disclosed. The cryoablation catheter includes: a vacuum zone provided therein with an inlet into which liquid coolant is injected and an outlet from which the liquid coolant discharged, wherein rest parts other than the inlet and the outlet therein are maintained in a vacuum state; a body part separated from the vacuum zone and provided at the front end thereof, wherein an end of the inlet and an end of the outlet communicate with each other, and wherein the body part includes a freeze zone for cooling surroundings by expanding of the liquid coolant exiting through a tip provided at the end of the inlet; and a tapered tip provided at an end of the body part, wherein a guidewire lumen for insertion of a guidewire thereinto is provided, and wherein the tapered tip is tapered toward an end thereof.

## Description

### CROSS REFERENCE TO RELATED APPLICATION

The present application claims priority to Korean Patent Application No. 10-2019-0001332, filed January 04, 2019, the entire contents of which is incorporated herein for all purposes by this reference.

### BACKGROUND OF THE INVENTION

### Field of the Invention

The present invention relates to a cryoablation catheter for treatment of hypertrophic cardiomyopathy and a method for treatment of hypertrophic cardiomyopathy using the same. More particularly, the present invention relates to a cryoablation catheter for treatment of hypertrophic cardiomyopathy and a method for treatment of hypertrophic cardiomyopathy using the same, wherein a cryoablation catheter is inserted into an hypertrophied myocardium, whereby a refrigerant is applied thereto so as to cause cold damage to the hypertrophied myocardium for the treatment of hypertrophic cardiomyopathy, a disease in which the interventricular septum of the left ventricle of the heart of an animal or human body becomes thick.

### Description of the Related Art

Hypertrophic cardiomyopathy is a heart disease in which the left ventricle wall thickens without having any symptom such as aortic valvular stenosis or high blood pressure that may cause left ventricular thickening. The disease is found in one in every 500 people, and medical diagnoses with regard to various types of the left ventricular thickening are observed. The most common and representative characteristics are asymmetrical septal hypertrophy and occlusion (i.e., obstruction caused by closing) of the left ventricular outflow tract with variability.

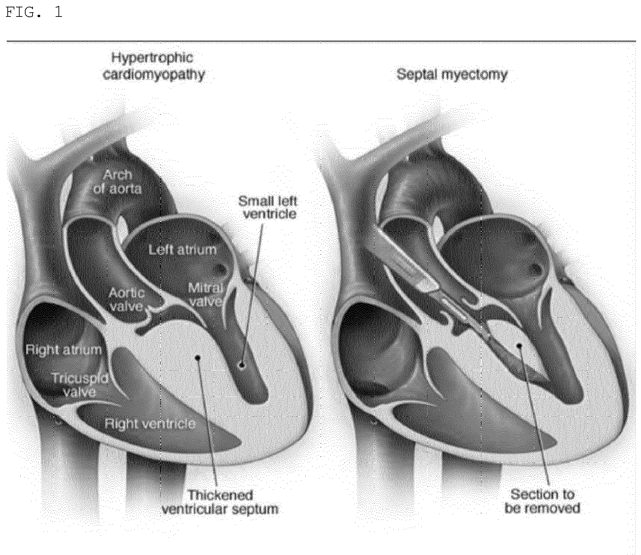

FIG. 1 is a diagram showing a symptom of hypertrophic cardiomyopathy and a current surgical method generally performed.

Referring to FIG. 1, the general surgical method of hypertrophic cardiomyopathy is performed by inserting a surgical knife through the aortic valve into the left ventricle and then cutting out the hypertrophied septum as shown. In other words, using the knife, a cardiologist performs a method of cutting out the hypertrophied septum in a professional sense.

Meanwhile, cryoablation is a technique for functionally ablating a tissue by cooling or freezing the tissue to a deadly extent. Cryoablation has a benefit of minimizing collateral tissue damage in the tissue which is required to be preserved, for example blood vessels and other tissues, while performing cryoablation. Also, cryoablation may be applied to extensive treatments including the treatments of cancer and heart disease.

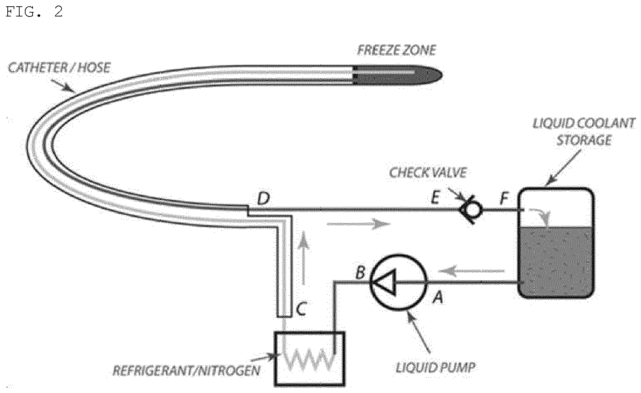

FIG. 2 is a diagram showing the basic structure of a conventional cryoablation system. A liquid coolant storage stores a low-temperature liquid coolant such as nitrogen, argon, or helium. The liquid coolant storage pumps the liquid coolant to a refrigerator through a liquid pump. The low-temperature liquid coolant from the refrigerator is injected into an inlet of a cryoablation catheter. A freeze zone is provided at the end of the cryoablation catheter. The low-temperature liquid coolant injected into the inlet rapidly expands in the freeze zone and quickly lowers the ambient temperature. This is due to the thermodynamic mechanism based on Joule-Thomson effect. An ice ball is generated in the human tissue around the freeze zone of the cryoablation catheter. The ice ball is meant to cause damage to the tissue, and may be verified through an image produced by ultrasound and the like. The liquid coolant expanded in the freeze zone is discharged from the cryoablation catheter again through an outlet, and is restored in the liquid coolant storage.

The cryoablation catheter is used as a very useful treatment tool in liver cancer and the like. However, the cryoablation catheter has not been widely used for the treatment of hypertrophic cardiomyopathy. In the case where a catheter tip of the conventional cryoablation catheter is inserted into the hypertrophied septum in a cavity in the left ventricle of the heart, a sufficient amount of therapeutic damage is not applied to the interventricular septum. This is undesirable because contact surface with ventricular septal tissue is insufficiently provided and the cooling effect is canceled by warm blood in the left ventricle, and thus this is in fact why conventional cryoablation is not widely applied for treatment of hypertrophic cardiomyopathy.

### Document of Related Art

(Patent Document 1) Korean Patent Application Publication No. 10-2016-0061394 (published on May 31, 2016)

### SUMMARY OF THE INVENTION

An objective of the present invention is to provide a soft catheter-type cryoablation catheter, wherein a tapered tip having a guidewire lumen at the end thereof is provided for treatment of hypertrophic cardiomyopathy, whereby a method for the treatment by cryoablation is provided with a therapeutic effect of intra-myocardial or intra-septal hypertrophic cardiomyopathy.

The objective of the present invention is not limited to the above-mentioned objective, and other objectives that are not mentioned will be clearly understood by those skilled in the art from the following description.

According to the present invention, there is provided a cryoablation catheter for treatment of hypertrophic cardiomyopathy, the cryoablation catheter including: a vacuum zone provided therein with an inlet into which liquid coolant is injected and an outlet from which the liquid coolant discharged, wherein rest parts other than the inlet and the outlet in the vacuum zone are maintained in a vacuum state; a body part separated from the vacuum zone and provided at the front end thereof, wherein an end of the inlet and an end of the outlet communicate with each other, and wherein the body part comprises a freeze zone for cooling surroundings by expanding of the liquid coolant exiting through a tip provided at the end of the inlet; and a tapered tip provided at an end of the body part, wherein a guidewire lumen for insertion of a guidewire thereinto is provided, and wherein the tapered tip is tapered toward an end thereof.

According to a preferred exemplary embodiment, the guidewire lumen may be in close contact with the guidewire without separation space.

According to the preferred exemplary embodiment, the guidewire lumen may be communicated from a tip hole, which is the end of the tapered tip, to a side hole which is provided on an opposite end side of the tapered tip.

According to the preferred exemplary embodiment, the tapered tip may be provided therein with a spiral-type coil wire or a braided-type wire.

According to the preferred exemplary embodiment, the tapered tip may be provided with a hydrophilic polymer coating on a surface thereof.

According to the preferred exemplary embodiment, the tapered tip may have a length of 5 to 20 mm, a tip of the tapered tip may have a thickness of 1.2 to 1.4 Fr, and the opposite side of the tip of the tapered tip may have a thickness of 3 to 6 Fr.

According to the preferred exemplary embodiment of the present invention, a method for treatment of hypertrophic cardiomyopathy using a cryoablation catheter, the method includes: i) placing a guidewire in a hypertrophied septum through coronary sinus and septal vein; ii) delivering the cryoablation catheter, which is provided with a guidewire lumen along the guidewire, to the hypertrophied septum; and iii) injecting liquid coolant into the cryoablation catheter and performing cryoablation on the hypertrophied septum in a cryoablation area.

According to the preferred exemplary embodiment, in performing the cryoablation, the cryoablation may be performed while monitoring an ice ball, which is formed around the cryoablation area, by using an imaging device.

According to the preferred exemplary embodiment, the guidewire lumen for insertion of the guidewire thereinto may be provided at an end of the cryoablation catheter, and wherein a tapered tip may be provided tapered toward an end thereof.

According to the present invention, there are the following effects:
1. In the present invention, since a freeze zone of the cryoablation catheter is inserted into the interventricular septum to perform cryoablation, the freeze zone does not directly contact the warm blood flow in the ventricles, and thus there is a benefit of sufficiently achieving the effect of the cryoablation.
2. The cryoablation catheter of the present invention has a tapered tip at the end thereof, and is inserted into the interventricular septum, along a guidewire, by using a guidewire lumen provided at a position inside the tapered tip, and thus there is a benefit of causing less tissue damage or encountering resistance when inserted into the interventricular septum.
3. There is a benefit in that an ice ball generated by the cryoablation catheter of the present invention may be visually verified. During treatment by cryoablation, while visually verifying the ice ball generated in the tissue through an image produced by ultrasound and the like, the treatment by cryoablation may be performed, thereby having a benefit of making a safer treatment possible.
4. Cryoablation does not cause severe pain in the patient. Cryoablation is a relatively painless myectomy compared to RF ablation, so patients generally keep a more stable state during the treatment.
5. There is no severe damage applied to large blood vessels. The vascular system is maintained in a normal state during cryoablation, and the structural function of the tissue is maintained stably even after finishing cryoablation.

### BRIEF DESCRIPTION OF THE DRAWINGS

FIG. 1 is a diagram showing a symptom of hypertrophic cardiomyopathy and a surgical method currently practiced in general.
FIG. 2 is a diagram showing the basic structure of a conventional cryoablation system.
FIG. 3 is a schematic diagram showing a cryoablation catheter for treatment of hypertrophic cardiomyopathy of the present invention and a method for the treatment using the same.
FIG. 4 is a schematic diagram of the cryoablation catheter for the treatment of hypertrophic cardiomyopathy of the present invention.

### DETAILED DESCRIPTION OF THE INVENTION

Advantages and features of the present invention and the methods of achieving the same will become apparent with reference to an exemplary embodiment described below in detail in conjunction with the accompanying drawings. However, the present invention is not limited to the exemplary embodiment disclosed below, but may be implemented in various different modes. The exemplary embodiment is provided only to make the disclosure of the present invention complete, and to fully convey the scope of the present invention to those skilled in the art. Therefore, the present invention is only defined by the scope of the claims.

The main point of the present invention is to provide a cryoablation catheter having a tapered tip at the end thereof, and provided with a guidewire lumen at the position inside the tapered tip. Also, the main point of the present invention is to provide a method for the treatment of intra-myocardial or intra-septal hypertrophic cardiomyopathy by cryoablation using this cryoablation cathether.

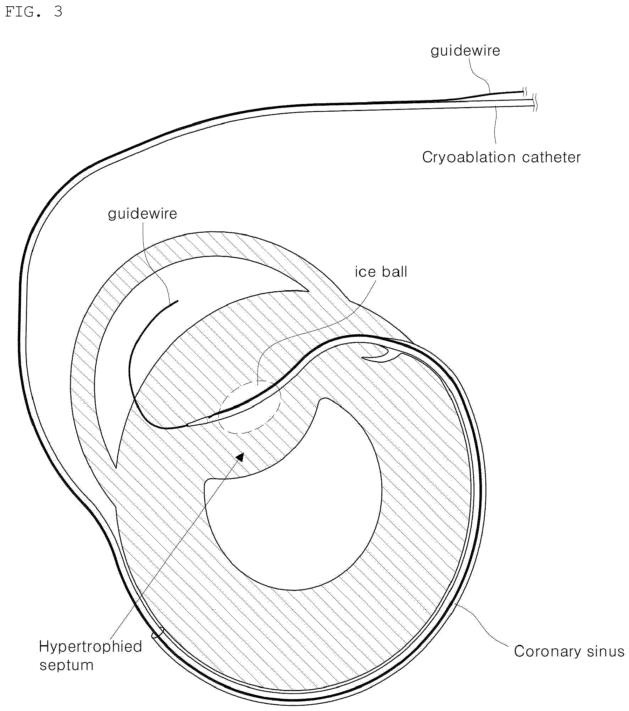

FIG. 3 is a schematic diagram showing the cryoablation catheter for the treatment of hypertrophic cardiomyopathy of the present invention and the method for the treatment using the same.

Referring to FIG. 3, in the present invention, the guidewire is positioned in the hypertrophied septum through the coronary sinus and septal vein, and then, along a pre-positioned guidewire, a freeze zone of the cryoablation catheter is reached at a target area (i.e., the hypertrophied septum) for the treatment. Then, liquid coolant is injected into an inlet from the outside, and cryoablation is performed by generating an ice ball around the freeze zone of the cryoablation catheter while the liquid coolant expands rapidly through a tip where the end of the inlet is provided.

First, a method of positioning the guidewire in the target area (i.e., the hypertrophied septum) for the treatment will be described.

A very thin guidewire (about 0.014") is used to pass through the coronary sinus and septal vein to be positioned in the hypertrophied septum. At this time, the position of the guidewire may be verified in real time by echocardiography and the like, thereby obtaining information for finding an optimal position.

In the present invention, the coronary sinus having an opening in the right atrium is used to access to the hypertrophied septum for the purpose of treating hypertrophic cardiomyopathy through the septal vein. Access to the coronary sinus is performed through the neck vein or the femoral vein, and a guiding catheter is used to access to the coronary sinus through the superior vena cava or the inferior vena cava.

The guiding catheter utilizes a balloon-tipped guiding catheter and/or a dual lumen microcatheter. The guiding catheter is a catheter for guiding the guidewire to a desired position.

After accessing to the coronary sinus, the guiding catheter is guided to a position where the distal part of the coronary sinus is, and a pressurized venogram is performed through the guiding catheter. The balloon-tipped guiding catheter is ideal for the pressurized venogram.

In the case where an image of the septal vein is visualized through the pressurized venogram, about 0.014" of PTCA guidewire is inserted into the septal vein closest to the target area. By the PTCA guidewire, a more precise treatment is possible by utilizing a real-time imaging device such as ultrasound (i.e., echocardiogram) that enables imaging guidance to indicate whether the guidewire is navigating to the target area. In order to more precisely guide the direction of the PTCA guidewire, a dual lumen microcatheter may be used to precisely guide the guidewire to the target area.

In other words, in order to position the guidewire in the interventricular septum through the septal vein, (1) the pressurized venogram using the balloon-tipped guiding cathether, and/or (2) the dual lumen microcatheter and the like may be used.

The pressurized venogram is used to take a better view of the septal vein. To this end, the balloon-tipped guiding catheter may be used as an auxiliary means.

In addition, the dual lumen microcatheter may be used as an auxiliary means in order to position the guidewire in the desired direction within the interventricular septum. The dual lumen microcatheter is a catheter provided with two lumens for inserting the guidewire. After the first guidewire is positioned in the septal vein through the first lumen of the dual lumen microcatheter, the second guidewire is inserted through the second lumen of the dual lumen microcatheter. The second guidewire may move in a different direction other than the direction of the first guidewire. As such, the dual lumen microcatheter is a very useful catheter assisting treatment when the guidewire navigates to the desired target position in the interventricular septum.

In the septal vein positioned in the ventricular septal tissue, other than the surface of the interventricular septum, the guidewire may be freely moved without causing a problem such as bleeding. Since the septal vein is present in the interventricular septum, the septal vein is used to position the guidewire into the hypertrophied intra-septum. When using the septal vein, the guidewire is possible to move to the desired position by breaking through the septal vein, as required. In this case, the dual lumen catheter described above may be used.

A similar approach is theoretically possible with the use of the coronary artery, but the coronary artery has various anatomical positions thereof, thereby often making it difficult to reach the target area. In this case, when the guidewire penetrates the coronary artery, serious problems such as intra-myocardial bleeding may occur. In addition, when the treatment is performed through the coronary artery, coronary thrombosis or coronary dissection often occurs in the coronary artery, and thus this may be fatal. Therefore, as in the present invention, the septal vein is used for insertion of the guidewire.

In the present invention, the approach through the septal vein may prevent the complications of the treatment, caused by the conventional approaches: an LV endocarium approach which passes through the coronary artery, the aortic valve, or the mitral valve; or an approach through direct needle puncture. Meanwhile, conventionally, there is a treatment using alcohol injection into the septal artery. Likewise, since this treatment is difficult to control the distribution of alcohol, the alcohol is delivered to the unwanted area, thereby causing problems such as unnecessary myocardial infarction and the like, and often causing a disease in the conduction system of the heart.

Next, the cryoablation catheter is positioned to the target area (i.e., the hypertrophied intra-septum) along the guidewire positioned at the hypertrophied septum. That is, one or more guidewires are positioned at the target position, and then the cryoablation catheter is positioned along the guidewire.

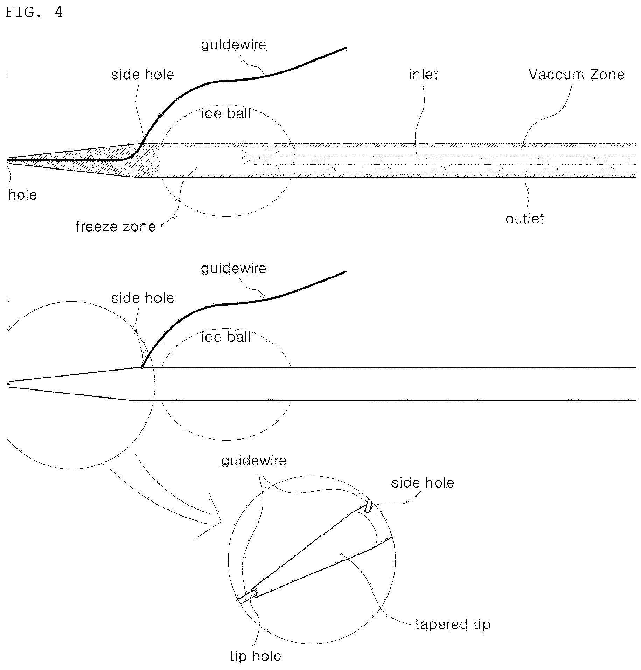

FIG. 4 is a schematic diagram of the cryoablation catheter for the treatment of hypertrophic cardiomyopathy according to the present invention.

The cryoablation catheter is divided into two parts: a body part, and a tapered tip.

The tapered tip is a part provided at the end of the body part, that is, at the end of the catheter. A guidewire lumen is provided in the tapered tip. The guidewire lumen has a thickness of about 3 to 6 Fr (i.e., preferably about 4 Fr), and this size gives less damage to the interventricular septum when inserted into the interventricular septum.

Preferably the length of the tapered tip is about 5 to 20 mm (i.e., preferably about 1 cm), and the end thereof has a thickness of about 1.2 to 1.4 Fr. The opposite side of the end thereof has the same thickness (i.e., preferably 3 to 6 Fr) as the contour of a septal insertion part. The tapered tip has the shape of a very long, thin tip that is 5 to 20 times longer than the thickness of the opposite side of the end thereof (i.e., the thickness of the contour of the body part).

The tapered tip has the guidewire lumen therein, so as to pass through the coronary sinus and the septal vein, and to navigate along the guidewire positioned in the interventricular septum. A tip hole is provided at the end of the tapered tip. The tip hole is connected to the guidewire lumen.

The guidewire lumen provided at a position inside the tapered tip must be in close contact with the guidewire without a separation space therebetween. The reason why there must be no space between the guidewire lumen of the tapered tip and the guidewire is that the tapered tip must minimize resistance without damaging the tissue of interventricular septum when inserted into the interventricular septum. In the present invention, this is because the tapered tip must penetrate the tissue of interventricular septum while functioning as a needle. In the case where the space is formed between the guidewire lumen and the guidewire, the tapered tip is resisted by the tissue of interventricular septum when inserted into the interventricular septum, thereby causing a possible problem of damaging the tissue of interventricular septum.

However, since the cryoablation catheter must be inserted into the interventricular septum along the guidewire, although the guidewire lumen of the tapered tip and the guidewire are in close contact, the cryoablation catheter must be able to move backward and forward along the guidewire.

In the present invention, the proximal part of the catheter means one end of the catheter where the catheter comes out of the human body, and the distal part of the catheter means the opposite end thereof where the cathether is inserted into the human body.

The tapered tip has stiffness harder than the body part. This is because the tapered tip is the part inserted into the tissue of interventricular septum.

Preferably, a spiral coil wire or braided wire is provided at a position in the tapered tip. This is to prevent the tapered tip from kinking during the insertion of the interventricular septum and to improve pushability and trackability. Pushability is the ability to transmit force from the proximal part to the distal part while reducing kinking, and trackability is the ability to inject the catheter through tortuous blood vasculature. In other words, pushability is often understood as the ability to transmit force from the proximal end of the catheter to the distal end of the catheter while minimizing or eliminating kinking. Trackability is often understood as the ability to navigate the catheter through tortuous vasculature.

Preferably, the tapered tip may be provided with a hydrophilic polymer coating on the surface thereof. This is to make the tapered tip easily moves into the tissue of interventricular septum. The hydrophilic polymer coating is harder than the soft catheter so that the tapered tip may be more easily inserted when inserted into the tissue of interventricular septum.

The body part is a part constituting the body of the catheter, and is divided into the freeze zone and a vacuum zone. The body part has the inlet through which the liquid coolant is injected and the outlet through which the liquid coolant is discharged.

The freeze zone is an area in which the liquid coolant of the inlet rapidly expands to form the ice ball around the freeze zone. The vacuum zone is an area in which the inlet and the outlet pass through, and the rest parts other than the inlet and the outlet in the vacuum zone maintain a vacuum state. The reason why the vacuum zone is kept in vacuum is to avoid damage to the tissue due to the low temperature of the liquid coolant passing through the inlet and the outlet.

Each end of the inlet and the outlet is open in the freeze zone. In the freeze zone, the cryoablation is performed while the liquid coolant expands through the tip provided at the end of the inlet. Since the inner diameter of the tip of the inlet is smaller than that of the inlet, the liquid coolant discharged through the tip expands instantaneously, thereby freezing the surroundings. The liquid coolant expanded in the freeze zone is discharged back to the outside through the outlet which is open.

The freeze zone and the vacuum zone of the body part have the same structure as the conventional cryoablation catheter in general, and the operation principle of the present invention is also the same as that of the conventional cryoablation catheter, and thus a detailed description thereof will be omitted.

In the present invention, when the septal insertion part is inserted into the interventricular septum, the body part must have sufficient stiffness to efficiently support the catheter to be inserted so that the catheter is prevented from kinking.

Meanwhile, the guidewire lumen provided at the position in the tapered tip has a structure composed between a tip hole as an entry side of the tapered tip and a side hole provided at the end of the opposite side of the tapered tip. This structure is called a "monorail type" or "rapid exchange type". The monorail type is distinguished from an over-the-wire type having a structure composed throughout the entire catheter starting from the tip hole. In the present invention, since the freeze zone and the vacuum zone are provided between the distal part and the proximal part of the catheter, the "over-the-wire type" catheter is difficult to be applied, and as shown, the monorail type catheter is applicable.

Cryoablation using the cryoablation catheter is performed several times until the position of the cryoablation catheter is moved slightly backward and forward along the guidewire, or until a therapeutic effect is achieved in various areas of the heart along other guidewires.

While being performed in the intra-myocardium (alternately, intra-septum), cryoablation may use an imaging device such as ultrasound in order to visually monitor, through verifying the ice ball phenomenon, the extent of tissue damage caused by cryoablation, thereby making a proper treatment possible.

Although the exemplary embodiment of the present invention has been described above with reference to the accompanying drawings, it may be understood that those skilled in the art to which the present invention pertains may implement the present invention in other specific forms without changing the technical spirit or essential features thereof. Therefore, a preferred exemplary embodiment of the present invention has been described for illustrative purposes, and should not be construed as being restrictive.

## Claims

1. A cryoablation catheter for treatment of hypertrophic cardiomyopathy, the cryoablation catheter comprising:
a vacuum zone provided therein with an inlet into which liquid coolant is injected and an outlet from which the liquid coolant discharged, wherein rest parts other than the inlet and the outlet in the vacuum zone are maintained in a vacuum state;
a body part separated from the vacuum zone and provided at the front end thereof, wherein an end of the inlet and an end of the outlet communicate with each other, and wherein the body part comprises a freeze zone for cooling surroundings by expanding of the liquid coolant exiting through a tip provided at the end of the inlet; and
a tapered tip provided at an end of the body part, wherein a guidewire lumen for insertion of a guidewire thereinto is provided, and wherein the tapered tip is tapered toward an end thereof.

2. The cryoablation catheter of claim 1, wherein the guidewire lumen is in close contact with the guidewire without separation space.

3. The cryoablation catheter of claim 1, wherein the guidewire lumen is communicated from a tip hole, which is the end of the tapered tip, to a side hole which is provided on an opposite end side of the tapered tip.

4. The cryoablation catheter of claim 1, wherein the tapered tip is provided therein with a spiral-type coil wire or a braided-type wire.

5. The cryoablation catheter of claim 1, wherein the tapered tip is provided with a hydrophilic polymer coating on a surface thereof.

6. The cryoablation catheter of claim 1, wherein the tapered tip has a length of 5 to 20 mm, a tip of the tapered tip has a thickness of 1.2 to 1.4 Fr, and the opposite side of the tip of the tapered tip has a thickness of 3 to 6 Fr.

7. A method for treatment of hypertrophic cardiomyopathy using a cryoablation catheter, the method comprising:
i) placing a guidewire in a hypertrophied septum through coronary sinus and septal vein;
ii) delivering the cryoablation catheter, which is provided with a guidewire lumen along the guidewire, to the hypertrophied septum; and
iii) injecting liquid coolant into the cryoablation catheter and performing cryoablation on the hypertrophied septum in a cryoablation area.

8. The method of claim 7, wherein, in performing the cryoablation, the cryoablation is performed while monitoring an ice ball, which is formed around the cryoablation area, by using an imaging device.

9. The method of claim 7, wherein the guidewire lumen for insertion of the guidewire thereinto is provided at an end of the cryoablation catheter, and wherein a tapered tip is provided tapered toward an end thereof.
